(19) Europäisches Patentamt
European Patent Office
Office européen des brevets

(11) Publication number: **0 046 242**
**B1**

(12) # EUROPEAN PATENT SPECIFICATION

(45) Date of publication of patent specification: **25.04.84**

(51) Int. Cl.³: **C 07 F 7/08**, A 61 K 31/695

(21) Application number: **81106147.2**

(22) Date of filing: **05.08.81**

(54) **Anti-tumor agent comprising an organosilicon compound.**

(30) Priority: **14.08.80 JP 112092/80**

(43) Date of publication of application:
**24.02.82 Bulletin 82/8**

(45) Publication of the grant of the patent:
**25.04.84 Bulletin 84/17**

(84) Designated Contracting States:
**CH DE FR GB LI**

(56) References cited:

**DERWENT SOVIET INVENTIONS ILLUSTRATED, vol. V, no. 21, 28th June 1974, Section I Chemical London, GB**
**DERWENT SOVIET INVENTIONS ILLUSTRATED, vol. V, no. 12, 30th April 1974, Section I Chemical London, GB**
**CHEMICAL ABSTRACTS, vol. 80, no. 3, 21st January 1974, page 440, abstract 14996t, Columbus, Ohio, US, M.G. VORONKOV et al.: "Aminoalkyl trialkylsilylalkyl sulfides"**

(73) Proprietor: **Shin-Etsu Chemical Co., Ltd.**
**6-1, Ohtemachi 2-chome**
**Chiyoda-ku, Tokyo (JP)**

(72) Inventor: **Toyoshima, Shigeshi**
**1-4-18, Naka-Meguro**
**Meguro-ku Tokyo (JP)**
Inventor: **Sato, Ryuichi**
**2310, Oaza-Kamikoizumi Oizumi-cho**
**Yura-gun Gunma-ken (JP)**
Inventor: **Ito, Koichi**
**4-3-32, Minami-cho**
**Higashi-kurume-shi Tokyo (JP)**
Inventor: **Shinohara, Toshio**
**3-19-1, Isobe Annaka-shi**
**Gunma-ken (JP)**
Inventor: **Yamamoto, Yasushi**
**992-4, Hachiman-cho Takasaki-shi**
**Gunma-ken (JP)**

(74) Representative: **Jaeger, Klaus, Dr. et al,**
**JAEGER & PARTNER Patentanwälte Bergstrasse 48 1/2**
**D-8035 München-Gauting (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 046 242 B1**

Courier Press, Leamington Spa, England.

# Anti-tumor agent comprising an organosilicon compound

## Background of the invention

The present invention relates to a novel anti-tumor agent comprising an organosilicon compound as the effective ingredient or, more particularly, to an anti-tumor agent comprising an $\omega$-(aminoalkylthio)alkyl trialkyl silane as the effective ingredient.

Needless to say, one of the subject matters of the highest interest in the field of pharmacology is to discover a compound having an activity as an anti-tumor agent or an anti-cancer agent, i.e. an activity to suppress or retard the growth of the tumor or cancer cells in a human body but having low toxicity to the body treated therewith. A great number of organic compounds have been proposed as effective as an anti-tumor agent and indeed some of them are used for therapeutical purposes even though not quite satisfactory in respect of effectiveness or toxicity to the patent's body.

Among the compounds tested for the anti-tumor activity, organic compounds containing a silicon atom in the molecule belong to one of the promising classes of compounds for the purpose. For example, silatrane compounds have been reported to be effective although the toxicity thereof prohibits the use of these compounds for the therapeutical purpose.

Several classes of organosilyl and organosilane compounds have been reported as effective as an anti-tumor agent including, for example, $\omega$-trialkylsilylalkanoic acids (see Japanese Patent Kokai 55—27133), bis(triorganosilylalkyl) phosphites (see Japanese Patent Kokai 55—45627 and U.S. Patent 4,267,172), $\omega$-triorganosilylalkanoic acid anilides (see Japanese Patent Kokai 55—83713) and 2-(3-trimethylsilylpropyl)benzothiazole (see Japanese Patent Kokai 56—43213).

These organosilyl compounds are effective as anti-tumor agents but the inventors have continued their investigations to discover more effective compounds with lower toxicity.

A method of preparing triorgano(dialkylaminomethylthio)alkansilanes is described in Derwent Soviet Inventions Illustrated, vol. V, no. 21, 28th June 1974, Section I Chemical, London, GB and SU—A—391,144 (M. G. Voronkov et al.), (15—11—1971).

Furthermore a method of preparing aminoalkyl(triorganylsilylalkyl)sulphides is described in Derwent Soviet Inventions Illustrated, vol. V, no. 12, 30th April, 1974, Section I Chemical, London, GB, and SU—A—386,948 (M. G. Voronkov et al.) (15—11—1971).

A method of preparing aminoalkyltrialkylsilylalkyl sulfides is described in Chemical Abstracts, vol. 80, no. 3, 21th January 1974, page 440, abstract 14996t, Columbus, Ohio, U.S. and by M. G. Voronkov et al.: "Aminoalkyl trialkylsilylalkyl sulfides" in Zh. Obshyh. Khim. 1973, 43(7), 1540—2.

## Summary of the invention

The anti-tumor agent of the present invention established as a result of the investigations of the inventors, of which the effectiveness has been confirmed by both the in vitro and in vivo tests, comprises an $\omega$-(aminoalkylthio)alkyl trihydrocarbyl silane of the general formula

$$R_3Si(CH_2)_mS(CH_2)_nNR^1_2, \qquad (I)$$

in which each of the groups R is independently a substituted or unsubstituted monovalent hydrocarbon group having from 1 to 6 carbon atoms, each of the groups $R^1$ is independently a hydrogen atom or an alkyl group having from 1 to 3 carbon atoms and m and n are each independently an integer from 1 to 6 inclusive, or a pharmacologically acceptable salt form thereof in a therapeutically effective amount. The compounds in the amine form are suitable for oral administration and the salt form thereof is suitable for both oral administration and administration by injection owing to the increased solubility in water. No particular toxicity was noted of the compounds at least in the animal test undertaken with mice as the test animals.

## Detailed description of the preferred embodiments

The $\omega$-(aminoalkylthio)alkyl trialkyl silanes of the general formula (I) as the effective ingredient of the inventive anti-tumor agent are not new as a class of the organosilicon compounds. They have been most intensively investigated by Russian investigators and some of them are reported in the Inventor's Certificates, USSR, Nos. 386948, 391144 and 514838, for example. These references disclose the synthetic methods for the preparation of the compounds.

The above references teach four types of the synthetic reactions, which are:

a) the addition reaction of a mercaptoalkyl-containing silane with an alkenyl amine according to the equation

$$R_3Si(CH_2)_mSH + CH_2{=}CH(CH_2)_{n-2}NR^1_2 \rightarrow R_3Si(CH_2)_mS(CH_2)_nNR^1_2; \qquad (II)$$

b) the addition reaction of an alkenyl-containing silane with a mercaptoalkyl amine according to the equation

$$R_3Si(CH_2)_{m-2}CH{=}CH_2 + HS(CH_2)_nNR^1_2 \rightarrow R_3Si(CH_2)_mS(CH_2)_nNR^1_2; \qquad (III)$$

c) the addition reaction of a mercaptoalkyl-containing silane with an ethyleneimine compound according to the equation

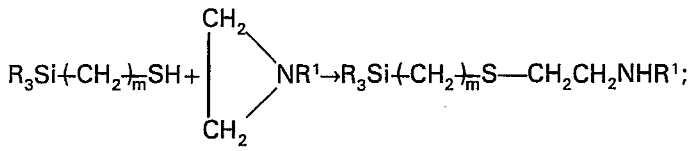 (IV)

and

d) the Mannich reaction of a mercaptoalkyl-containing silane with paraformaldehyde as the source of formaldehyde according to the equation

$$R_3Si(CH_2)_mSH + CH_2O + HNR^1_2 \rightarrow R_3Si(CH_2)_mS—CH_2—NR^1_2, \quad (V)$$

in which R, $R^1$, m and n each have the same meaning as defined above.

Similar methods to the above described reactions b) and c) are also described in U.S. Patent 3,660,454.

In the above given general formula (I), the hydrocarbon group denoted by R has from 1 to 6 carbon atoms and is exemplified by alkyl groups such as methyl, ethyl and propyl groups, alkenyl groups such as vinyl group and aryl groups such as phenyl group. The group R may be a substituted hydrocarbon group obtained by partial substitution of the hydrogen atoms in the above named hydrocarbon groups with halogen atoms or cyano groups as exemplified by trifluoropropyl group. The alkyl group denoted by $R^1$ is exemplified by methyl, ethyl and propyl groups. The integers denoted by m and n are each from 1 to 6 inclusive but preferably from 1 to 3, since these compounds are easier to synthesize.

Several of the examples in conformity with the above described definition of the general formula (I) and the symbols R, $R^1$, m and n are as follows, in which Me, Et, Vi and Ph each denote a methyl, ethyl, vinyl or phenyl group, respectively.

$Et_3Si(CH_2)_2S(CH_2)_2NH_2$

$Ph\ Me_2\ Si(CH_2)_2S(CH_2)_2NH_2$

$Vi\ Me_2\ Si(CH_2)_2S(CH_2)_2NH_2$

$(CF_3CH_2CH_2)\ Me_2\ Si(CH_2)_2S(CH_2)_2NH_2$

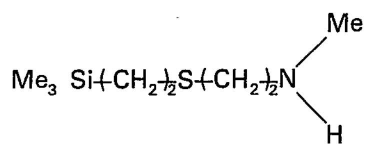

$Me_3\ Si(CH_2)_2S(CH_2)_2NMe_2$

$Me_3\ Si(CH_2)_2S(CH_2)_2NEt_2$

$Me_3\ Si(CH_2)_2S(CH_2)_2NH_2$

$Et_3Si(CH_2)_3S(CH_2)_2NEt_2$

$Me_3\ Si(CH_2)_3S(CH_2)_3NH_2$

$Me_3\ Si(CH_2)_2S—CH_2NEt_2$

$Me\ Et_2\ Si(CH_2)_2S—CH_2NEt_2$

$Me_3\ Si(CH_2)_2S—CH_2NEt_2$

$Et_3\ Si(CH_2)_2S—CH_2NEt_2$

$Me_3\ Si(CH_2)_2S(CH_2)_3NH_2$

$Me_3\ Si(CH_2)_2S(CH_2)_3NEt_2$

$Me_3\ Si\text{+}CH_2\text{+}_3S—CH_2NEt_2$

$Et_3\ Si\text{+}CH_2\text{+}_3S—CH_2NEt_2$

$Me\ Et_2\ Si—CH_2—S—CH_2NEt_2$

$Et_3\ Si—CH_2—S—CH_2NEt_2$

$Et_3\ Si—CH_2—S\text{+}CH_2\text{+}_2NH_2$

As a matter of due course in the experimentation, the inventors have first synthesized the particular compounds disclosed in the above mentioned Russian references and undertaken the animal tests of these compounds for the anti-tumor activity with mice as the test animals. When the compounds were used as such in the amine form, the compound was blended with the feed and, when the compound had been converted to the salt form, e.g. a salt with hydrochloric acid or acetic acid, the test was undertaken by injecting the aqueous solution of the salt subcutaneously utilizing the increased solubility of the compound in water along with the oral administration as reported hereunder. The results were that all of the compounds tested were more or less effective in suppressing the growth of the B—16 melanoma cells, some of them being remarkably more effective than others. Meanwhile, it was noted that the administration of the compound in the amine form by the injection method was relatively ineffective indicating that the effectiveness of the compound is exhibited only when it is in a water-soluble salt form accessible to the tumor cells with the assumption that the compound of the amine form orally administrated is readily converted to the salt form in the acidic medium in the stomach.

Furthermore the investigations were directed to the synthesis of several unknown compounds not disclosed in the above Russian references, though belonging to the class of the ω-(aminoalkylthio)alkyl trihydrocarbylsilane compounds, and the screening test of these newly synthesized compounds for the anti-tumor activity. The novel compounds synthesized by the inventors include 2-(2-aminoethylthio)ethyl trimethyl silane, 2-(2-aminoethylthio)ethyl phenyl dimethyl silane, 2-(2-aminoethylthio)ethyl 3,3,3-trifluoropropyl dimethyl silane, 2-(2-aminoethylthio)ethyl vinyl dimethyl silane as well as the hydrochlorides and acetates thereof. The most promising compound among them is the first mentioned 2-(2-aminoethylthio)ethyl trimethyl silane of the formula

$$(CH_3)_3Si\text{+}CH_2\text{+}_2S\text{+}CH_2\text{+}_2NH_2$$

and the hydrochloride or acetate thereof.

This particular compound could be synthesized by any one of the above described synthetic methods a), b) and c), of which best results were obtained by the method b) according to the equation (III) utilizing the photochemical addition reaction between vinyl trimethyl silane and 2-mercaptoethylamine as the starting reactants.

In the following, the results of the synthesis and identification of this compound and the results of the in vitro and in vivo tests for the anti-tumor activity of this compound along with some other compounds of the same class including both known ones reported in the references and the newly synthesized ones.

Example 1

Into a glass flask of 1 liter capacity equipped with a thermometer, a reflux condenser and a highpressure mercury lamp of 100 watts power and mounted on a magnetic stirrer unit were introduced 41 g (0.41 mole) of vinyl trimethyl silane, 30.8 g (0.40 mole) of 2-mercaptoethylamine and 2 g of benzophenone as the photosensitizer and dissolved by adding 300 ml of tetrahydrofuran and 20 ml of water to give a reaction mixture. The photochemical reaction was carried out at 25°C with agitation under a nitrogen atmosphere by irradiating with the light emitted from the mercury lamp.

As the reaction proceeded, the 2-mercaptoethylamine remaining at first partly undissolved became dissolved to give a clear reaction mixture. The proceeding of the reaction was followed by the gas chromatographic analysis for the contents of the unreacted reactants and the ultraviolet irradiation was terminated when the content of the unreacted 2-mercaptoethylamine had decreased to 5% or less of the starting content. The reaction was usually complete within 8 hours of irradiation though dependent on the intensity of the light.

Distillation of the above obtained reaction mixture under reduced pressure gave 56.7 g of a fraction boiling at 83—85°C under a pressure of 4 mm Hg (5 mbar) having a refractive index $n_D^{25}$ of 1.4821.

The analytical results given below supported that the above obtained compound was the desired 2-(2-aminoethylthio)ethyl trimethyl silane. The yield of the compound was about 80% of the theoretical value.

Infrared absorption spectral analysis:
3260 to 3370 $cm^{-1}$ ($—NH_2$); and
1250 $cm^{-1}$ and 840 to 860 $cm^{-1}$ ($—Si(CH_3)_3$)

Nuclear magnetic resonance spectral analysis: (Measurements were made in 10% $CCl_4$ solution with the $—Si(CH_3)_3$ of the compound per se as the internal reference):

0 p.p.m. $—Si(CH_3)_3$, singlet, 9H;
0.70 to 0.90 p.p.m. $—Si—CH_2—$, multiplet, 2H;
1.58 to 1.74 p.p.m. $—NH_2$, 2H;
2.35 to 2.60 p.p.m. $—CH_2—S—CH_2—$, multiplet, 4H; and
2.60 to 2.82 p.p.m. $—N—CH_2—$, multiplet, 2H

Elementary analysis

| | Found, % | Calculated as $C_7H_{19}NSSi$, % |
|---|---|---|
| C | 47.2 | 47.4 |
| H | 10.7 | 10.8 |
| N | 8.0 | 7.9 |
| S | 18.3 | 18.1 |
| Si | 15.8 | 15.8 |

The above prepared 2-(2-aminoethylthio)ethyl trimethyl silane can be readily converted to the hydrochloride or acetate. For example, the hydrochloride was prepared in a manner described below.

Thus, 10.0 g (0.056 mole) of the silane compound and 50 ml of n-hexane were introduced into a flask of 100 ml capacity equipped with a thermometer, a reflux condenser and a gas inlet tube and anhydrous hydrogen chloride gas was blown into the mixture kept at 10 to 30°C at a rate of 30 to 50 ml/minute. The reaction mixture turned yellow with some temperature elevation with subsequent appearance of white precipitates. When more than the stoichiometric amount of the hydrogen chloride gas had been blown into the mixture, the precipitates were dissolved to again give a clear, colorless solution where the heat evolution ceased and the hydrogen chloride gas was no longer absorbed in the reaction mixture.

The above obtained reaction mixture was stripped of the excess of hydrogen chloride and n-hexane under reduced pressure to leave a white solid in an amount of 11.3 g which was dried overnight in a desiccator. This product was very hygroscopic, had a melting point of 74 to 76°C and was identified to be the desired hydrochloride of 2-(2-aminoethylthio)ethyl trimethyl silane from the results of the infrared absorption spectral analysis and the elementary analysis. The above given yield was about 93.7% of the theoretical value.

The acetate, instead of hydrochloride above obtained, was also readily obtained in a needle-like crystalline form having high hygroscopicity by adding acetic acid into the reaction mixture instead of blowing of hydrogen chloride gas.

Example 2

The vinyl trimethyl silane used in Example 1 was replaced with an equimolar amount of vinyl triethyl silane, vinyl phenyl dimethyl silane or allyl trimethyl silane and the reaction in each case was conducted substantially in the same manner as in Example 1 to give 2-(2-aminoethylthio)ethyl triethyl silane, 2-(2-aminoethylthio)ethyl phenyl dimethyl silane or 3-(2-aminoethylthio)propyl trimethyl silane, respectively.

Example 3

Into a solution of 2.41 g of mercaptomethyl triethyl silane in 30 ml of methyl alcohol was added a solution of 0.65 g of ethyleneimine in 10 ml of methyl alcohol under nitrogen atmosphere and the reaction mixture was heated for 4 hours at 55 to 60°C. After completion of the reaction, the reaction mixture was stripped of the solvent and subjected to distillation under reduced pressure to give a fraction boiling at 127 to 128°C under a pressure of 3 mm Hg (4 mbar) and having a refractive index $n_D^{20}$ of 1.4998. This product was identified to be (2-aminoethylthio)methyl triethyl silane.

Example 4

An in vitro test by tissue culture was undertaken for the activity of 2-(2-aminoethylthio)ethyl trimethyl silane against the growth of three kinds of tumor cells. The tumor cells used in the test were Ehrlich's cancer cells, Lewis lung cancer cells and B—16 melanoma cells and each kind of the above tumor cells was cultured in several Eagle's nutrient media admixed with 10% of calf blood plasma at 37°C.

After 24 hours from the start of the culture, 2-(2-aminoethylthio)ethyl trimethyl silane was added

to each of the nutrient media in a different concentration and, after additional three days at the same temperature, the concentration $IC_{50}$ was determined with which the multiplication of the tumor cells was inhibited in 50% of the tested groups. The results were as shown below

| | $IC_{50}$, $\gamma$/ml |
|---|---|
| Ehrlich's cancer cells | 12 |
| Lewis lung cancer cells | 14 |
| B—16 melanoma cells | 18 |

Example 5

Animal tests were conducted to examine the activity of several compounds including 2-(2-amino-ethylthio)ethyl trimethyl silane in the inhibiting power against the growth of the B—16 melanoma cells implanted in mice as the test animals.

Thus each of 12 female mice of the $BDF_1$—JCR lineage was implanted subcutaneously with $1\times10^5$ cells of the B—16 black tumor.

Six of the thus treated mice were bred as such as a control group and the other six mice as the test group were orally administrated once a day with 100 mg of the test compound for 5 days beginning with the second day of the implantation.

Fourteen days after the implantation of the tumor cells, all of the mice were killed and the weights of the tumors were determined. The results of the tests were expressed by the tumor inhibiting efficiency in % calculated by the following equation, in which $W^1$ is the averaged tumor weight per mouse in the control group and $W^2$ is the averaged tumor weight per mouse in the test group.

Tumor inhibiting efficiency,

$$\% = \frac{W^1 - W^2}{W^1} \times 100$$

For comparison, similar animal tests were undertaken with FT—207 (N-tetrahydrofuryl-5-fluorouracil) with the dose amount at one time increased to 130 mg.

The results of the above animal tests were summarized in Table 1 below, in which the data of the boiling point and the refractive index of each of the tested compounds are reported along with the results of the tumor inhibiting efficiency. In the table, the symbols Me, Et, Vi and Ph each have the same meaning as given above.

TABLE 1

| Compound | Boiling point (°C) / at pressure (mbar) | Refractive index, $n_D^{20}$ | Tumor inhibiting efficiency (%) |
|---|---|---|---|
| $Me_3Si{+}CH_2{)_2}S{+}CH_2{)_2}NH_2$ | 83—85/5.3 | 1.4823 | 95.6 |
| $Me_3Si{+}CH_2{)_2}S{+}CH_2{)_2}NH_2 \cdot HCl$ | — | — | 97.9 |
| $Me_3Si{+}CH_2{)_2}S{+}CH_2{)_2}NH_2 \cdot CH_3COOH$ | — | — | 95.0 |
| $Et_3Si{+}CH_2{)_2}S{+}CH_2{)_2}NH_2$ | 135—137/1.3 | 1.4232 | 90.8 |
| $Ph \cdot Me_2Si{+}CH_2{)_2}S{+}CH_2{)_2}NH_2$ | 165—167/2.7 | 1.5412 | 82.6 |
| $Vi \cdot Me_2Si{+}CH_2{)_2}S{+}CH_2{)_2}NH_2$ | 96—98/5.3 | — | 95.7 |
| $(CF_3CH_2CH_2)Me_2Si{+}CH_2{)_2}S{+}CH_2{)_2}NH_2$ | 111—115/2.7 | — | 96.8 |
| $Me_3Si{+}CH_2{)_3}S{+}CH_2{)_2}NH_2$ | 88—90/2.7 | — | 93.9 |
| $Et_3Si{+}CH_2{)_3}S{+}CH_2{)_2}NEt_2$ | 172—175/5.3 | 1.4809 | 76.7 |
| $Me_3Si{+}CH_2{)_2}S{-}CH_2{-}NEt_2$ | 100—101/5.3 | 1.4726 | 89.2 |
| $Me \cdot Et_2Si{+}CH_2{)_2}S{-}CH_2{-}NEt_2$ | 150—152/5.3 | 1.4782 | 86.9 |
| $Et_3Si{+}CH_2{)_2}S{-}CH_2{-}NEt_2$ | 130—132/6.7 | 1.4840 | 90.5 |
| $Me_3Si{+}CH_2{)_3}S{-}CH_2{-}NEt_2$ | 104—105/4.0 | — | 83.9 |
| $Et_3Si{+}CH_2{)_3}S{-}CH_2{-}NEt_2$ | 156—157/4.0 | 1.4820 | 84.4 |
| $Me \cdot Et_2Si{-}CH_2{-}S{-}CH_2{-}NEt_2$ | 108—110/5.3 | 1.4764 | 86.5 |
| $Et_3Si{-}CH_2{-}S{-}CH_2{-}NEt_2$ | 106—108/5.3 | 1.4800 | 81.7 |
| $Et_3Si{-}CH_2{-}S{+}CH_2{)_2}NH_2$ | 127—128/4.0 | 1.4798 | 91.1 |
| FT—207 | — | — | 20.5 |

## Claims

1. An anti-tumor agent comprising, as the effective ingredient thereof, an ω-(aminoalkylthio)alkyl trihydrocarbyl silane represented by the general formula

$$R_3Si{+}CH_2{)_m}S{+}CH_2{)_n}NR^1{}_2,$$

in which each of the groups R is independently a substituted or unsubstituted monovalent hydrocarbon group having from 1 to 6 carbon atoms, each of the groups $R^1$ is independently a hydrogen atom or an alkyl group having from 1 to 3 carbon atoms and m and n are each independently an integer from 1 to 6, or a pharmacologically acceptable salt form thereof in a therapeutically effective amount.

2. The anti-tumor agent as claimed in claim 1 wherein the unsubstituted hydrocarbon group denoted by R is selected from the class consisting of methyl, ethyl, propyl, vinyl and phenyl groups.

3. The anti-tumor agent as claimed in claim 1 wherein the substituted hydrocarbon group denoted by R is 3,3,3-trifluoropropyl group.

4. The anti-tumor agent as claimed in claim 1 wherein the alkyl group denoted by $R^1$ is selected from the class consisting of methyl, ethyl and propyl groups.

5. The anti-tumor agent as claimed in claim 1 wherein m and n are each independently an integer from 1 to 3.

6. The anti-tumor agent as claimed in claim 1 wherein the ω-(aminoalkylthio)alkyl trihydrocarbyl silane is 2-(2-aminoethylthio)ethyl trimethyl silane.

7. The anti-tumor agent as claimed in claim 1 wherein the salt form is the hydrochloride or acetate.

8. 2-(2-Aminoethylthio)ethyl trimethyl silane.

**Patentansprüche**

1. Antitumormittel, enthaltend als Wirkkomponente ein ω-(Aminoalkylthio)-alkyl-trikohlenwasserstoff-silan der allgemeinen Formel

$$R_3Si\text{-}(CH_2)_m\text{-}S\text{-}(CH_2)_n\text{-}NR^1_2$$

in der jeder der Reste R unabhängig ein substituierter oder unsubstituierter einwertiger Kohlenwasserstoffrest mit 1 bis 6 Kohlenstoffatomen, jeder der Reste $R^1$ unabhängig ein Wasserstoffatom oder eine Alkylgruppe mit 1 bis 3 Kohlenstoffatomen und m und n jeweils unabhängig voneinander eine ganze Zahl von 1 bis 6 sind, oder ein pharmakologisch unbedenkliches Salz dieses Silans in therapeutisch wirksamer Menge.

2. Antitumormittel nach Anspruch 1, dadurch gekennzeichnet, daß der unsubstituierte Kohlenwasserstoffrest R Methyl, Ethyl, Propyl, Vinyl oder Phenyl ist.

3. Antitumormittel nach Anspruch 1, dadurch gekennzeichnet, daß der substituierte Kohlenwasserstoffrest R 3,3,3-Trifluorpropyl ist.

4. Antitumormittel nach Anspruch 1, dadurch gekennzeichnet, daß der Alkylrest $R^1$ Methyl, Ethyl oder Propyl ist.

5. Antitumormittel nach Anspruch 1, dadurch gekennzeichnet, daß m und n unabhängig voneinander jeweils eine ganze Zahl im Bereich von 1 bis 3 einschließlich sind.

6. Antitumormittel nach Anspruch 1 dadurch gekennzeichnet, daß das ω-(Aminoalkylthio)-alkyl-trikohlenwasserstoffsilan das 2-(2-Aminoethylthio)-ethyl-trimethylsilan ist.

7. Antitumormittel nach Anspruch 1, dadurch gekennzeichnet, daß das Salz das Hydrochlorid oder das Acetat ist.

8. 2-(2-Aminoethylthio)-ethyl-trimethylsilan.

**Revendications**

1. Agent anti-tumeur contenant comme substance active an ω-(aminoalkylthio)alkyl-trihydrocarbylsilane de formule générale:

$$R_3Si\text{-}(CH_2)_m\text{-}S\text{-}(CH_2)_n\text{-}NR^1_2$$

dans laquelle chaque groupe R est, indépendamment des autres, un groupe hydrocarbure monovalent substitué ou non substitué ayant de 1 à 6 atomes de carbone, chaque groupe $R^1$ est, indépendamment de l'autre un atome d'hydrogène ou un groupe alkyle ayant de 1 à 3 atomes de carbone et m et n sont, indépendamment l'un de l'autre, un nombre entier compris entre 1 et 6, ou bien un sel pharmacologiquement acceptable de ce composé en une quantité thérapeutiquement active.

2. Agent anti-tumeur selon la revendication 1, caractérisé en ce que le groupe hydrocarbure non substitué R est choisi parmi les groupes méthyle, éthyle, propyle, vinyle et phényle.

3. Agent anti-tumeur selon la revendication 1, caractérisé en ce que le groupe hydrocarbure non substitué R est le groupe 3,3,3-trifluoropropyle.

4. Agent anti-tumeur selon la revendication 1, caractérisé en ce que le groupe alkyle désigné par $R^1$ est choisi parmi les groupes méthyle, éthyle et propyle.

5. Agent anti-tumeur selon la revendication 1, caractérisé en ce que m et n sont chacun l'un indépendamment de l'autre, un nombre entier compris entre 1 et 3.

6. Agent anti-tumeur selon la revendication 1, caractérisé en ce que l'ω-(amino-alkylthio)alkyl-trihydrocarbylsilane est le 2-(2-amino-éthyl-thio)éthyl-triméthylsilane.

7. Agent anti-tumeur selon la revendication 1, caractérisé en ce que le sel est le chlorhydrate ou l'acétate.

8. Le 2-(2-amino-éthylthio)éthyl-triméthylsilane.